(19) Europäisches Patentamt
European Patent Office
Office européen des brevets

(11) Publication number: **0 180 745**
**A1**

# (12) EUROPEAN PATENT APPLICATION

(21) Application number: 85111879.4

(22) Date of filing: 11.02.83

(51) Int. Cl.⁴: C 07 C 123/00
C 07 C 101/34, C 07 C 69/716
C 07 C 69/738, C 07 D 239/36
C 07 D 213/55, C 07 D 231/12
C 07 D 235/16, C 07 D 215/14
C 07 D 317/60, C 07 D 317/62

**A request pursuant to Rule 88 EPC for correction of the claims was filed on 29.10.1985.**

(30) Priority: 15.02.82 JP 22280/82
26.08.82 JP 148123/82
27.08.82 JP 148967/82
01.09.82 JP 151997/82
13.12.82 JP 218209/82

(43) Date of publication of application:
14.05.86 Bulletin 86/20

(84) Designated Contracting States:
AT BE CH DE FR GB IT LI LU NL SE

(60) Publication number of the earlier application in accordance with Art. 76 EPC: 0 086 647

(71) Applicant: YAMANOUCHI PHARMACEUTICAL CO. LTD.
No. 5-1 Nihonbashi-Honcho, 2-chome Chuo-ku
Tokyo(JP)

(72) Inventor: Yanagisawa, Isao
No. 2-22-8, Shakujiidai Nerima-ku
Tokyo(JP)

(72) Inventor: Ohta, Mitsuaki
No. 3-16-1, Hasune Itabashi-ku
Tokyo(JP)

(72) Inventor: Takagi, Tokuichi
No. 8-27-13, Takasago Katsushika-ku
Tokyo(JP)

(74) Representative: Geering, Keith Edwin et al,
REDDIE & GROSE 16 Theobalds Road
London WC1X 8PL(GB)

## (54) Acylacetate and amidine intermediates for pyrimidone compounds, and their preparation.

(57) Compounds of formulae II and III below are useful as intermediates for the preparation of pyrimidones of formula I below which are gastric acid secretion inhibitors and have other medical utility:

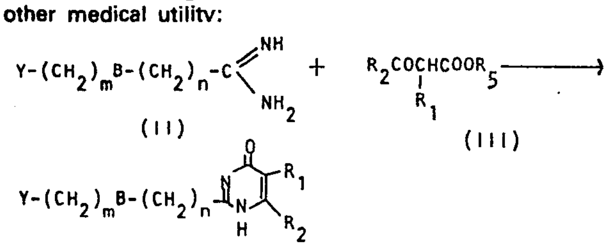

(II) (III)

(I)

wherein $R_1$ represents a hydrogen atom, an alkyl group, a lower alkenyl group, a lower alkinyl group, a phenyl group, a cycloalkylmethyl group, an unsubstituted or substituted aralkyl group, or the group

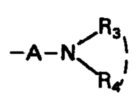

wherein A represents a lower alkylene group and $R_3$ and $R_4$ are the same or different lower alkyl groups or combine to form with the adjacent nitrogen atom a piperidine or pyrrolidine ring; $R_2$ represents a hydrogen atom, a lower alkyl group or a trifluoromethyl group; Y represents a heterocyclic group of the formula:

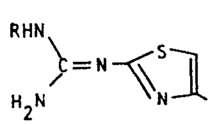

,

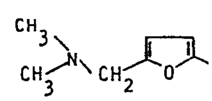

or

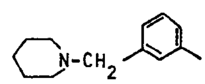

wherein R represents a hydrogen atom or a lower alkyl group; B represents an oxygen atom or a sulfur atom; m represents zero or an integer of 1 to 3; and n represents an integer of 1 to 3.

EP 0 180 745 A1

# ACYLACETATE AND AMIDINE INTERMEDIATES FOR PYRIMIDONE COMPOUNDS, AND THEIR PREPARATION

The present invention relates to intermediates for the preparation of pyrimidone compounds which are useful in general as gastric acid secretion inhibitors and some of which are also useful as anti-inflammatory agents or medicaments for treating disease of the cardio-vascular system or disease of the stomach. The invention also relates to preparation of these intermediates.

Known pyrimidone compounds include those of the formula :

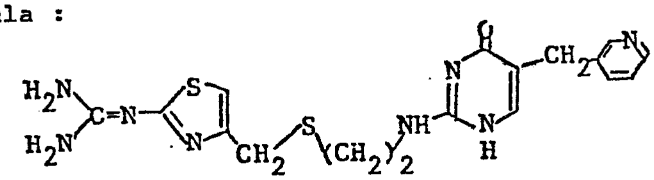

which exhibit prolonged gastric acid secretion inhibiting activity (published Japanese Patent Application No: 55-115883); and those of the formula :

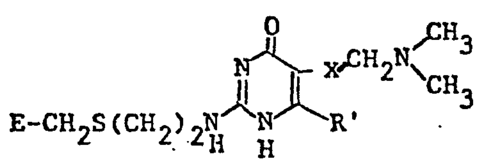

where E is

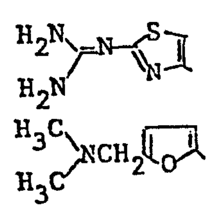

or

and X is $-CH_2$-heterocyclic- ,

which have the characteristic of a dimethylaminomethyl group at the 5-position. Other pyrimidone derivatives

are disclosed in published Japanese patent applications Nos. 54-115385 and 55-500898.

EP 0 086 647 A discloses pyrimidone compounds of general formula I :

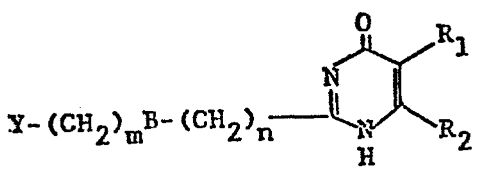

I

wherein $R_1$ represents a hydrogen atom, an alkyl group, a lower alkenyl group, a lower alkinyl group, a phenyl group, a cycloalkylmethyl group, an unsubstituted or substituted aralkyl group, or the group 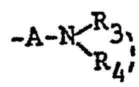 wherein A represents a lower alkylene group and $R_3$ and $R_4$ are the same or different lower alkyl groups or combine to form with the adjacent nitrogen atom a piperidine or pyrrolidine ring; $R_2$ represents a hydrogen atom, a lower alkyl group or a trifluoromethyl group; Y represents a heterocyclic group of the formula :

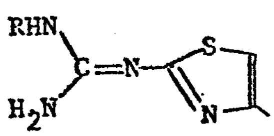 , 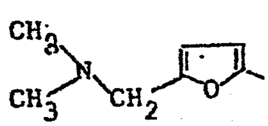 or 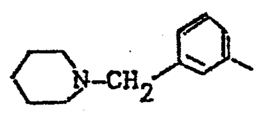

wherein R represents a hydrogen atom or a lower alkyl group; B represents an oxygen atom or a sulfur atom; m represents zero or an integer of 1 to 3; and n represents an integer of 1 to 3; and the pharmacologically acceptable salts thereof.

The term "lower" in the above definition means a straight or branched carbon chain having 1 to 5 carbon atoms. For example, as lower alkyl groups, there are methyl, ethyl, propyl, isopropyl, butyl, isobutyl, sec-bytyl, tert-butyl, and pentyl groups etc; as lower alkenyl groups there are vinyl and allyl groups etc; as lower alkinyl groups there are ethinyl, 2-propinyl, 3-butinyl, 2-butinyl, 1-butinyl, 1-methyl-2-propinyl, and pentinyl groups etc.; and as lower alkylene groups there are methylene, ethylene, methylmethylene, trimethylene, propylene, tetramethylene, methyltrimethylene, and pentamethylene groups etc.

The alkyl group in the definition of $R_1$ is a straight or branched alkyl group having 1 to 10 carbon atoms. For example, as the alkyl group, there are, in addition to the above listed lower alkyl groups, n-hexyl, isohexyl, 1-methylpentyl, 2-methylpentyl, 3-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,2,2-trimethylpropyl, 1-ethyl-2-methylpropyl, 1-ethyl-1-methylpropyl,

n-heptyl, 1-methylhexyl, 2-methylhexyl, 3-methylhexyl, 4-methylhexyl, 5-methylhexyl, 1,1-dimethylpentyl, 1,2-dimethylpentyl, 1,3-dimethylpentyl, 1,4,dimethylpentyl, 2,2-dimethylpentyl, 2,3-dimethylpnentyl, 2,4-dimethylpentyl, 1-ethlylpentyl, 2-ethylpentyl, 3-ethylpentyl, 1-ethyl-1-methylbutyl, 1-ethyl-2-methylbutyl, 1-ethyl-3-methylbutyl, 2-ethyl-1-methylbutyl, 2-ethyl-2-methylbutyl, 1,1,2-trimethylbutyl, 1,2,2-trimethylbutyl, 1,2,3-trimethylbutyl, 1-propylbutyl, n-octyl, 6-methylheptyl, n-nonyl, 7-methyloctyl, n-desyl, and 8-methylnonyl groups, etc.

When $R_1$ is an aralky group, the aryl portion may for example be a phenyl or naphthyl group, a 5 or 6 membered heterocyclic group containing nitrogen atom(s) such as a pyridyl, pyrazolyl or imidazolyl group etc., or other unsaturated heterocyclic group containing nitrogen atom(s) such as a benzimidazolyl, indazolyl or quinolyl group etc, Such aryl groups may have one or more substituents such as lower alkyl, halogen, lower alkoxy, lower alkylenediosy, phenyl etc. As such halogens there are fluorine, chlorine, bromine etc.; as such alkoxy groups, there are for example methoxy, ethoxy, propoxy and butoxy groups etc.; and as such lower alkylenedioxy groups there are for example methylenedioxy and ethylenedioxy groups etc.

As the above aryl group, there are particularly listed naphthyl, pyridyl, benzimidazolyl, and phenyl optionally substituted by one or more substituent(s) selected from halogens and tert-butyl, methylenedioxy and phenyl groups.

As cycloalky groups herein there are for example cyclohexyl and cyclopentyl groups etc.

Typical compounds of formula I are :

5-(p-tert-butylbenzyl)-2-[2-[[[2-[(diaminomethylene)amino]-4thiazolyl]methyl]thio]ethyl]-6-methyl-4(lH)-pyrimidone;

2-[2-[[[2-[(diaminomethylene)amino]-4-thiazolyl]methyl]thio]-ethyl]-5-n-butyl-6-methyl-4(lH)-pyrimidone;

2-[2-[[[2-[(diaminomethylene)amino]-4-thiazolyl]methyl]thio]-ethyl]-6-methyl-5-n-pentyl-4(lH)-pyrimidone;

2-[2-[[[2-[(diaminomethylene)amino]-4-thiazolyl]methyl]thio]-ethyl]-6-methyl-5-(l-naphthylmethyl)-4(lH)-pyrimidone;

2-[2-[[[2-[(diaminomethylene)amino]-4-thiazolyl]methyl]thio]-ethyl]-5-(3-dimethylaminopropyl)-6-methyl-4(lH)-pyrimidone;

2-[2-[[[2-[(diaminomethylene)amino]-4-thiazolyl]methyl]-thio]ethyl]-5-(3-diethylaminopropyl)-6-methyl-4(lH)-pyrimidone;

5-[(1,3-benzodioxole-5-yl)methyl]-2-[2-[5-(dimethylamino-methyl)furfurylthio]ethyl]-6-methyl-4(lH)-pyrimidone; and

5-(p-tert-butylbenzyl)-6-methyl-2-[3-(m-piperidinomethyl-phenoxy)propyl]-4(lH)-pyrimidone.

The compounds of formula I can form acid addition salts, with mineral acids such as hydrochloric acid, hydrobromic acid, hydroiodic acid and sulfuric acid, etc., and with organic acids such as maleic acid, fumaric acid, and picric acid, etc. Furthermore, there also exist tautomers of the compounds of formula I at the 1H- and 3H-positions, and there exist occasionally hydroxy-tautomers in a small proportion.

The compounds of formula I and their acid addition salts have histamine $H_2$-receptor blocking activity, and are useful as gastric acid secretion inhibitors; some additionally have histamine $H_1$-receptor blocking activity and are useful also as anti-inflammatory agents and as medicaments acting on the cardio-vascular system.

Further, some compounds of formula I have cytoprotective action, and such compounds are useful as medicaments, for treating various diseases of the stomach.

For many of the compounds of formula I their activities, such as histamine $H_2$-receptor blocking activity and/or histamine $H_1$-receptor blocking activity, are prolonged, lasting for a long period of time.

Most at least of the compounds of formula I wherein $R_1$ is an alkyl group having 4 to 9 carbon atoms posses not only histamine $H_2$-receptor blocking activity but also cytoprotective action, and may therefore be useful as medicaments for treating various stomach diseases.

The intermediate compounds of the present invention are some of those of formulae II and III below, which can be used for preparation of formula I compounds as follows :

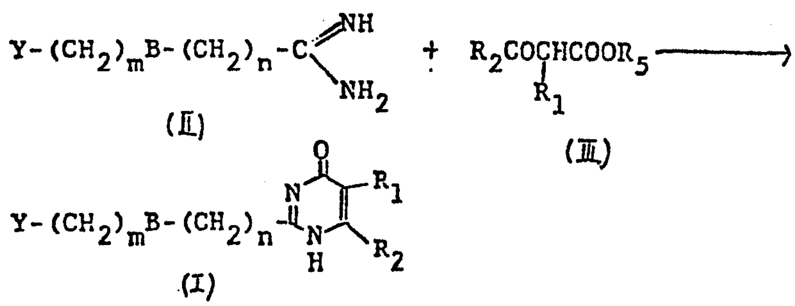

(II) (III)

(I)

wherein $R_5$ represents a lower alkyl group and the other symbols are as defined above.

This process is a condensation cyclization reaction between the amidine compound of formula (II) and the acylacetic acid derivative of formula (III).

The reaction is usually performed in a solvent at room temperature or under heating. As the solvent, there is preferably used alcohol (e.g. methanol, ethanol, isopropanol, etc), dimethylformamide, dimethylsulfoxide, methylcellosolve, ethylcellosolve, diglyme, etc. If necessary, a basic material may be present, suitable base materials including alkali metal (e.g. sodium or potassium) alcoholate, sodium hydroxide etc.

The starting materials (II) and (III) may be used in equimolar amounts, or some excess of either may be used.

The compounds of formula I thus prepared can be separated as the free base or an acid addition salt thereof, and further can be purified by standard procedures such as column chromatography, recrystallization, etc.

The starting materials of formula II can be obtained as disclosed in published Japanese patent applications Nos.55-115860, 55-115877 and 55-118476. The novel starting materials of formulae II and III, which constitute the compounds of the present invention, include those of Examples 1 to 44 below.

The use of these compounds to prepare formula I compounds, and the use and pharmaceutical activity of formula I compounds, are described and exemplified in EP 0 086 647 A.

In the following Examples, which illustrate preparation of compounds according to the invention, mp., Anal., NMR and Mass. are abbreviations for melting point, elementary analysis values, nuclear magnetic resonance spectrum and mass spectrum, respectively.

Example 1

Ethyl α-(3-pyridylmethyl)acetoacetate

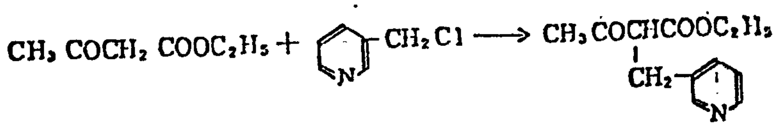

Under nitrogen, 8.4 g of metallic sodium was dissolved in 500 ml of anhydrous ethanol, and after adding 23.8 g of ethyl acetoacetate thereto and stirring the solution at room temperature for 1 hour, 30 g of 3-chloromethylpyridine hydrochloride was added to the solution followed by heating mildly for 20 hours. After filtering away the salt formed, the solvent was distilled off under reduced pressure, and the residue formed was purified by column chromatography using chloroform - ethyl acetate as the developing solvent to provide 12.0 g of the oily product.

NMR (in $CDCl_3$)

| | | |
|---|---|---|
| δ (ppm) ; | 1.18 | (t, 3H) |
| | 2.20 | (s, 3H) |
| | 3.13 | (d, 2H) |
| | 3.78 | (t, 1H) |
| | 4.13 | (q, 2H) |
| | 7.04～7.60 | (m, 2H) |
| | 8.40 | (m, 2H) |

Mass. (m/z) 221 ($M^+$)

Examples 2 - 18

By following the same procedure as in Example 1, the following compounds were obtained:

## Example 2

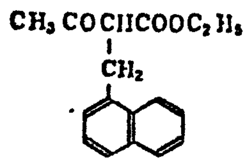

ethyl α-(1-naphthylmethyl)acetoacetate

NMR (in $CDCl_3$)

δ(ppm); 1.16 (t, 3H)
2.15 (s, 3H)
3.64 (d, 2H)
3.95 (t, 1H)
4.13 (q, 2H)
7.2~8.2 (m, 7H)

Mass(m/z) 270($M^+$), 227, 181

## Example 3

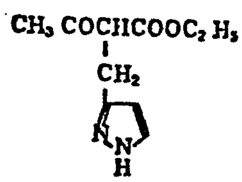

ethyl α-(3-pyrazolylmethyl)acetoacetate

NMR (in $CDCl_3$)

δ(ppm); 1.24 (t, 3H)
2.24 (s, 3H)
3.24 (d, 2H)
3.94 (t, 1H)
4.19 (q, 2H)
6.10 (d, 1H)
7.50 (d, 1H)

Mass(m/z) 210($M^+$), 167

Example 4

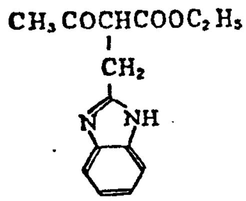

ethyl α-(2-benzimidazolylmethyl)-acetoacetate

Mass (m/z) 260 ($M^+$), 217

Example 5

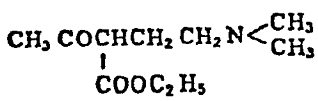

ethyl α-(2-N,N-dimethyl-aminoethyl)acetoacetate

NMR ($CDCl_3$)

| | | |
|---|---|---|
| δ (ppm); | 1.28 | (t, 3H) |
| | 2.08 | (q, 2H) |
| | 2.1~2.4 | (11H) |
| | 3.58 | (t, 1H) |
| | 4.20 | (q, 2H) |

Mass (m/z) 201 ($M^+$), 156

Example 6

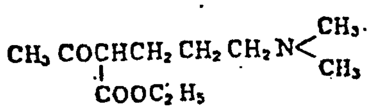

ethyl α-(3-N,N-dimethyl-aminopropyl)acetoacetate

NMR ($CDCl_3$)

| | | |
|---|---|---|
| δ (ppm); | 1.28 | (t, 3H) |
| | 1.4~1.6 | (m, 2H) |
| | 1.7~2.0 | (m, 2H) |
| | 2.1~2.5 | (11H) |
| | 3.44 | (t, 1H) |
| | 4.19 | (q, 2H) |

Mass (m/z) 215 ($M^+$), 170

Example 7

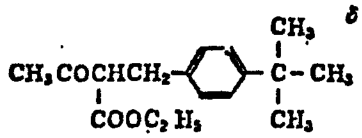

ethyl α-(p-tert-butylbenzyl)acetoacetate

NMR ($CDCl_3$)

δ (ppm) ; 1.18 (t, 3H)
1.27 (s, 9H)
2.17 (s, 3H)
3.11 (d, 2H)
3.75 (t, 1H)
4.14 (q, 2H)
7.08 (d, 2H)
7.28 (d, 2H)
7.00~7.36 (m, 4H)

IR (neat)

ν ; 1735, 1710 $cm^{-1}$

Mass (m/z)

276 ($M^+$), 261 ($M^+$-15)
233 ($M^+$-43)

Example 8

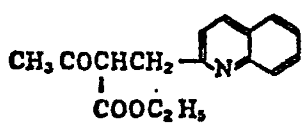

ethyl α-(2-quinolylmethyl)-acetoacetate

NMR ($CDCl_3$)

δ (ppm) ; 1.26 (t, 3H)
2.45 (s, 3H)
3.50~3.67 (m, 2H)
4.20 (q, 2H)
4.54 (t, 1H)
7.20~8.08 (m, 6H)

IR (neat)

ν ; 1730, 1705 $cm^{-1}$

Example 9

Mass (m/z)

271 ($M^+$), 256 ($M^+$-15)

228 ($M^+$- 43)

ethyl α-(p-chlorobenzyl)acetoacetate

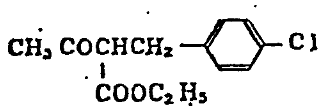

NMR ($CDCl_3$)

δ (ppm) ; 1.18 (t, 3H)

2.17 (s, 3H)

3.08 (d, 2H)

3.70 (t, 1H)

4.10 (q, 2H)

6.90~7.45 (m, 4H)

IR (neat)

ν ; 1735, 1710 $cm^{-1}$

Mass (m/z)

254 ($M^+$), 211 ($M^+$-43)

Example 10

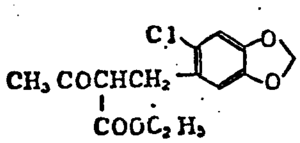

ethyl α-[(6-chloro-1,3-benzodioxole-5-yl)methyl]-acetoacetate

NMR ($CDCl_3$)

δ (ppm) ; 1.20 (t, 3H)

2.18 (s, 3H)

2.94~3.35 (m, 2H)

3.60~3.87 (m, 1H)

4.10 (q, 2H)

5.83 (s, 2H)

6.54~6.81 (m, 2H)

IR (neat)

ν ; 1735, 1710 $cm^{-1}$

Example 11.

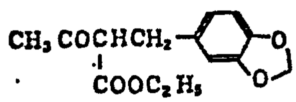

ethyl α-[(1,3-benzodioxole-5-yl)methyl]-acetoacetate

NMR($CDCl_3$)
δ(ppm) ; 1.20 (t, 3H)
2.16 (s, 3H)
3.05 (d, 2H)
3.68 (t, 1H)
4.10 (q, 2H)
5.82 (s, 2H)
6.58 (s, 3H)

IR(neat)
ν ; 1730, 1710 $cm^{-1}$

Mass (m/z)
264 ($M^+$), 191 ($M^+$-73)

Example 12

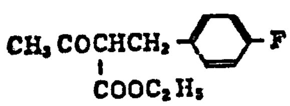

ethyl α-(p-fluorobenzyl)acetoacetate

Mass (m/z) 234 ($M^+$)
IR(neat) ν ; 1700 $cm^{-1}$
NMR($CDCl_3$)
δ(ppm) ; 1.22 (t, 3H)
2.18 (s, 3H)
2.30 (s, 3H)
3.16 (d, 2H)
3.70 (dt, 1H)
4.14 (q, 2H)
7.04 (s, 4H)

Example 13

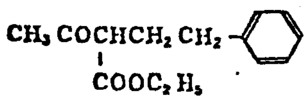

ethyl α-phenethyl-acetoacetate

Mass (m/z) 235 ($M^+$+1)
NMR ($CDCl_3$ )
δ (ppm) ; 1.25 ( t, 3H)
2.10~2.75 (m, 4H)
2.20 ( s, 3H)
3.35 ( t, 1H)
4.20 ( q, 2H)
7.15 ( s, 5H)

Example 14

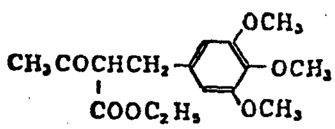

ethyl α-[(3,4,5-trimethoxy)benzyl]acetoacetate

Mass (m/z) 310 ($M^+$)
NMR ($CDCl_3$ )
δ (ppm) ; 1.22 ( t, 3H)
2.20 ( s, 3H)
3.12 ( d, 2H)
3.6~3.95 (m, 10H)
4.18 ( q, 2H)
6.40 ( s, 2H)

Example 15

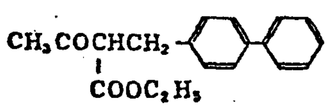

ethyl α-[(p-phenyl)-benzyl]acetoacetatc

Mass (m/z) 224 ($M^+$-COOEt+1)
IR (neat) ν ; 1710 $cm^{-1}$
NMR ($CDCl_3$ )
δ (ppm) ; 1.22 ( t, 3H)
2.20 ( s, 3H)
3.20 ( d, 2H)
3.78 ( dt, 1H)
4.18 ( q, 2H)
7.10~7.65 (m, 9H)

Example 16

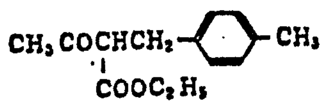

ethyl α-(p-methylbenzyl)acetoacetate

Mass (m/z) 234 ($M^+$)
IR (neat) ν ; 1700 $cm^{-1}$
NMR ($CDCl_3$)
δ (ppm) ; 1.22 (t, 3H)
2.18 (s, 3H)
2.30 (s, 3H)
3.16 (d, 2H)
3.70 (dt, 1H)
4.14 (q, 2H)
7.04 (s, 4H)

Example 17

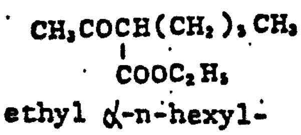

ethyl α-n-hexylacetoacetate

Mass (m/z) 214 ($M^+$)
NMR ($CDCl_3$)
δ (ppm) ; 0.80~2.05 (m, 16H)
2.20 (s, 3H)
3.35 (t, 1H)
4.15 (q, 2H)

Example 18

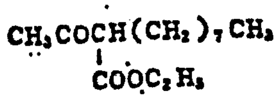

ethyl α-n-octylacetoacetate

Mass (m/z) 242 ($M^+$)
NMR ($CDCl_3$)
δ (ppm) ; 0.80~2.00 (m, 20H)
2.20 (s, 3H)
3.35 (t, 1H)
4.20 (q, 2H)

## Example 19

Ethyl formacetate

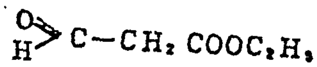

Under nitrogen, 0.23 g of chipped metallic sodium was dissolved in 10 ml of dry ether; while stirring vigorously, a solution of 0.88 g of ethyl acetate and 0.81 g of ethyl formate in 5 ml of dry ether was added dropwise to the mixture maintained at a temperature below -10$^{o}$C for 3 hours, and the stirring was continued for 2 hours under these conditions. Thereafter, the mixture was stirred at room temperature for 1 day, and the solvent was distilled off under reduced pressure to provide the unpurified oily product.

## Example 20

By following the same procedure as in Example 19, the following compounds were obtained.

| | | |
|---|---|---|
| (1) 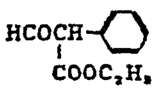 | (2) 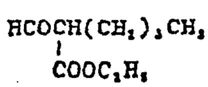 | (3) 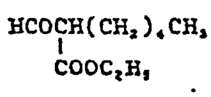 |
| ethyl α-phenyl-formacetate | ethyl α-n-butyl-formacetate | ethyl α-n-pentyl formacetate |

Examples 21 - 30

By following the same procedure as in Example 1, the following compounds were obtained:

Example 21

$CH_3COCHCH_2CH_2CH_2N<^{C_2H_5}_{C_2H_5}$ b.p. 110~114℃/1.8 mmHg
$COOC_2H_5$ Mass (m/z) 243 ($M^+$)
198 ($M^+-45$)

ethyl α-(3-diethylaminopropyl)acetoacetate

Example 22

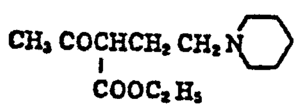

b.p.. 125~130℃/2.0 mmHg
Mass (m/z) 241 ($M^+$)
196 ($M^+-45$)

ethyl α-(2-piperidinoethyl)acetoacetate.

Example 23

$CH_3COCHCH_2CH_3$ b.p. 110℃/25 mmHg
$COOC_2H_5$ Mass (m/z) 159 ($M^+ +1$)

ethyl α-ethylacetoacetate

Example 24

$CH_3COCHCH_2CH_2CH_3$
$\quad\quad|$
$\quad COOC_2H_5$

ethyl α-propyl-acetoacetate

b.p. 125～130℃/25mmHg

Mass (m/z) 173 ($M^+$+1)

Example 25

$CH_3COCHCH_2C{\equiv}CH$
$\quad\quad|$
$\quad COOC_2H_5$

ethyl α-(2-propinyl)-acetoacetate

b.p. 135～140℃/25mmHg

Mass (m/z) 169 ($M^+$+1)

Example 26

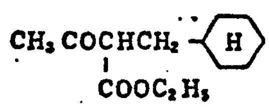

ethyl α-cyclohexyl-methylacetoacetate

Mass (m/z) 227 ($M^+$+1)

NMR ($CDCl_3$ )

δ (ppm) ; 0.8～1.90 (m, 13H)
1.25 (t, 3H)
2.30 (s, 3H)
3.45 (t, 1H)
4.15 (q, 2H)

Example 27

$CH_3COCH(CH_2)_3CH_3$
$\quad\quad|$
$\quad COOC_2H_5$

ethyl α-n-butylaceto-acetate

Mass (m/z) 187 ($M^+$+1)

NMR ($CDCl_3$ )

δ (ppm) ; 0.75～2.05 (m, 9H)
1.25 (t, 3H)
3.35 (t, 1H)
4.15 (q, 2H)

Example 28

$CH_3COCH(CH_2)_4CH_3$
$\quad\quad |$
$\quad\quad COOC_2H_5$

ethyl α-n-pentyl-
acetoacetate

Mass (m/z) 201 ($M^+$+1)
NMR ($CDCl_3$)
δ (ppm) ; 0.70~2.05 (m, 1H)
1.25 (t, 3H)
3.35 (t, 1H)
4.15 (q, 2H)

Example 29

$CH_3COCHCH_2CH_2CH<^{CH_3}_{CH_3}$
$\quad\quad |$
$\quad\quad COOC_2H_5$

ethyl α-[(3-methyl)-
butyl]acetoacetate

Mass (m/z) 201 ($M^+$+1)
NMR ($CDCl_3$)
δ (ppm) ; 0.90 (d, 6H)
1.05~2.05 (m, 5H)
1.25 (t, 3H)
3.30 (t, 1H)
4.15 (q, 2H)

Example 30

$\quad\quad\quad\quad\quad\quad CH_3$
$\quad\quad\quad\quad\quad\quad |$
$CH_3COCHCH_2CHCH_2N<^{CH_3}_{CH_3}$
$\quad\quad |$
$\quad\quad COOC_2H_5$

ethyl α-[3-(N,N-dimethyl-
amino)-2-methylpropyl]-
acetoacetate

b.p. 75~78℃/0.4 mmHg
Mass (m/z) 229 ($M^+$)

Example 31

(1)

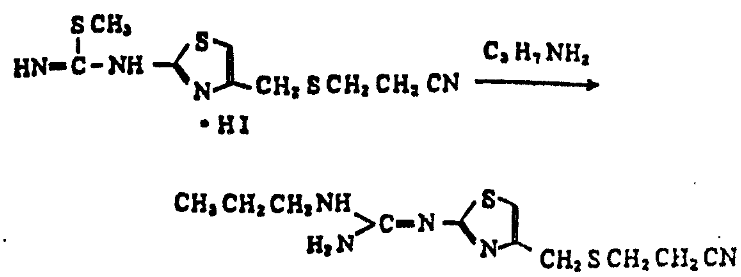


8.75 g of 3-[[[2-(S-methylisothioureido)thiazole-4-yl]methyl]thio]propionitrile hydroiodide and 12.9 g of n-propylamine were dissolved in 200 ml of ethanol, and the solution was refluxed under heating for 48 hours. The solvent was distilled away, and the residue formed was purified by column chromatography using a mixture of chloroform and methanol as the developing solvent to provide 6.0 g of 3-(2-n-propylguanidinothiazol-4-ylmethylthio)propionitrile

NMR ($CDCl_3$)

δ (ppm) ; 1.03 (t, 3H)

1.68 (m, 2H)

2.4 – 2.9 (m, 4H)

3.20 (t, 2H)

3.72 (s, 2H)

6.44 (s, 1H)

Mass (m/z) 283 ($M^+$)

(2) 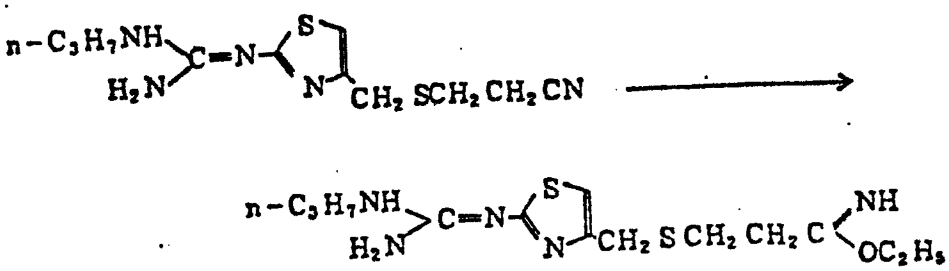

6.0 g of the nitrile compound obtained as in (1) above was dissolved in 100 ml of dry chloroform and after adding 20 ml of dry ethanol thereto and cooling the solution below 10°C and passing therethrough dry hydrogen chloride gas for 3 hours, the solution was allowed to stand at 0-4°C for 48 hours. Then, the solvents were distilled off and the residue was poured into a cooled aqueous solution of potassium carbonate for alkalifying, and the mixture was extracted with chloroform. The chloroform layer was washed with water and dried over anhydrous magnesium sulfate, and the solvent was distilled off under reduced pressure to provide 6.0 g of the oily product.

(3) 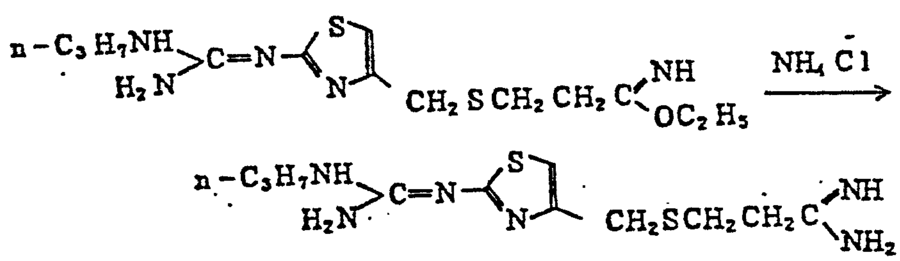


A mixture of 6.0 g of the imidate compound obtained as at (2) above and 1.0 g of ammonium chloride in 70 ml of methanol was stirred at room temperature for 1 hour, and the solvent was distilled off under reduced pressure. The residue was treated with hydrochloric acid in ethanol, and was recrystallized with acetonitrile - ethanol to provide 5.8 g of the amidine hydrochloride having a melting point of 183 - 184.5°C.

Example 32

4-(m-piperidinomethylphenoxy) butaneamidine hydrochloride

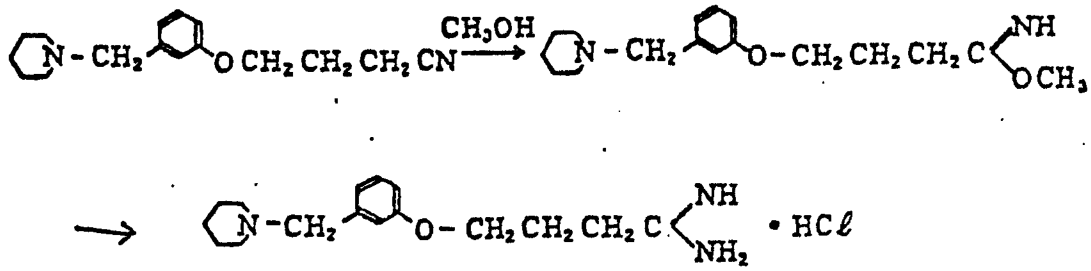


To 200 ml of dry chloroform were added 20.0 g (77.4 ml) of 4-(m-piperidinomethylphenoxy)butanenitrile and 15.7 ml (387 mmol) of dry methanol, and the solution obtained was cooled by ice-water bath. Through the solution was passed hydrogen chloride gas at 10°C. After confirming the disappearance of the starting material in 8 hours, the reaction was stopped, and the solvent was distilled off under reduced pressure. The residue was dissolved in chloroform, and the solution was added to saturated aqueous potassium carbonate solution and stirred. The organic solvent layer was separated, and the aqueous layer was extracted with chlorform. The organic layer and the chloroform solutionwere combined, and the combined solution was dried over anhydrous magnesium sulfate. The solvent was distilled away to provide oily crude methyl 4-(m-piperidinomethyl-phenoxy)butaneimidate (21.1 g). The product was dissolved in 400 ml of dry methanol, and 3.5 g (65.4 mmol) of ammonium chloride was added to the obtained solution at room temperature. The solution became homogeneous in 10 minutes, and completion of the reaction was checked by thin layer chromatography. The imidate disappeared after stirring the solution for 3 hours (checked by chromatography). A small amount of precipitate was filtered away and the solution was concentrated to precipitate crystals. The precipitated crystals were collected by filtration to provide 12.3 g of colourless needle crystals.

The filtrate was concentrated to dryness. By recrystallization from ethyl acetate-ethanol, 5.6 g of product was obtained (18.0 mmol, 23%).

m.p. 151.0 - 152.0 °C (ethanol-ethyl acetate)

Mass. (m/e) 275 ($M^+$- HCl)

Elemental analysis for $C_{16}H_{25}N_3O$ HCl

| | C | H | N | Cl |
|---|---|---|---|---|
| Calculated | 61.62 | 8.40 | 13.47 | 11.37 |
| Found | 61.47 | 8.51 | 13.52 | 11.41 |

Example 33

Ethyl α-(3-pyridylmethyl)acetoacetate

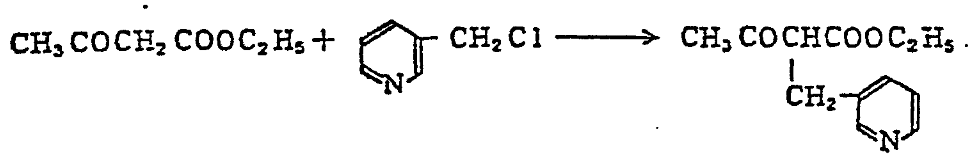

In 500 ml of anhydrous ethanol was dissolved 8.4 g of metallic sodium and after adding thereto 23.8 g of acetoacetate, the mixture was stirred for 1 hour at room temperature. Then, 30 g of 3-chloromethylpyridine hydrochloride was added to the mixture, and the mixture obtained was refluxed under mild heating. The salt formed was filtered away and the solvent was then distilled off under reduced pressure. The residue was purified by column chromatography using chloroform - ethyl acetate as a developing solvent to provide 12.0 g of oily product.

NMR ($CDCl_3$)

| | | |
|---|---|---|
| δ(ppm) ; | 1.18 | (3H, t) |
| | 2.20 | (3H, s) |
| | 3.13 | (2H, d) |
| | 3.78 | (1H, t) |
| | 4.13 | (2H, q) |
| | 7.04～7.60 | (2H, m) |
| | 8.40 | (2H, m) |

Mass(m/e) 221 ($M^+$)

Examples 34-38

By following the same procedure as in EXample 33, the following compounds were obtained:

Example 34

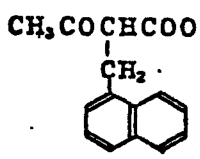

ethyl α-(1-naphythylmethyl)acetoacetate

NMR ($CDCl_3$)

| | | |
|---|---|---|
| δ(ppm) ; | 1.16 | (3H, t) |
| | 2.15 | (3H, s) |
| | 3.64 | (2H, d) |
| | 3.95 | (1H, t) |
| | 4.13 | (2H, q) |
| | 7.2～8.2 | (7H, m) |

Mass(m/e) 270($M^+$), 227, 181

Example 35

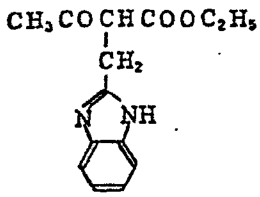

ethyl α-(2-benzimidazolyl-
methyl)acetoacetate

Mass (m/e) 260 ($M^+$), 217

Example 36

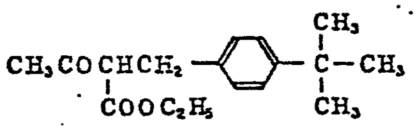

ethyl α-(p-tert-butylbenzyl)-
acetoacetate

NMR ($CDCl_3$ 中)
δ (ppm) ; 1.18 (t, 3H)
1.27 (s, 9H)
2.17 (s, 3H)
3.11 (d, 2H)
3.75 (t, 1H)
4.14 (q, 2H)
7.08 (d, 2H)
7.28 (d, 2H)
7.00~7.36 (m, 4H)

IR (neat)
ν ; 1735, 1710 $cm^{-1}$

Mass (m/e)
276 ($M^+$), 261 ($M^+-15$)
233 ($M^+-43$)

Example 37

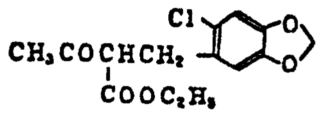

ethyl α- (6-chloro-1,3-benzodioxole-5-yl)methyl - acetoacetate

NMR ( $CDCl_3$ )

δ(ppm) ; 1.20 ( t, 3H )

2.18 ( s, 3H )

2.94~3.35 ( m, 2H )

3.60~3.87 ( m, 1H )

4.10 ( q, 2H )

5.83 ( s, 2H )

6.54~6.81 ( m, 2H )

IR ( neat )

ν ; 1735, 1710 $cm^{-1}$

Mass ( m/e )

298 ($M^+$), 255 ($M^+-43$)

Example 38

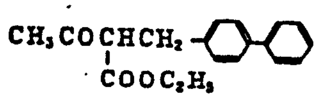

ethyl. α-(p-phenylbenzyl)-acetoacetate

NMR ( $CDCl_3$ )

δ(ppm) ; 1.22 ( t, 3H )

2.20 ( s, 3H )

3.20 ( d, 2H )

3.78 ( dt, 1H )

4.18 ( q, 2H )

7.10~7.65 ( m, 9H )

IR ( neat )

ν ; 1710 $cm^{-1}$

Mass ( m/e )

224 ($M^+-COOC_2H_5+1$)

Example 39

3-[5-(dimethylaminomethyl)furfurylthiomethyl] propionamidine di hydrochloride

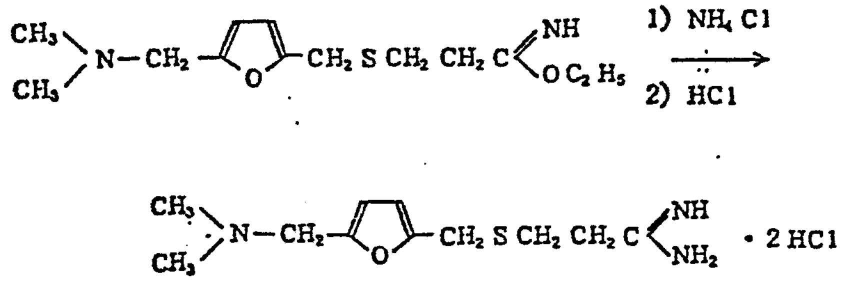


34 g of ethyl 3- 5-(dimethylaminomethyl)furfurylthiomethyl propionimidate was dissolved in 250 ml of dry methanol, and after adding 6 g of ammonium chloride and stirring the mixture at room temperature for 1 hour, an ethanol solution containing hydrochloric acid was added to the mixture to produce a dihydrochloride, and the solvent was distilled off. The residue was recrystallized from a mixture of isopropanol and ethyl acetate to provide 3- 5-(dimethylaminomethyl)furfurylthiomethyl propionamidine di-hydrochloride (31 g) having a melting point of 134 - 136°C.

Example 40

Ethyl α-(3-pyridylmethyl)acetoacetate

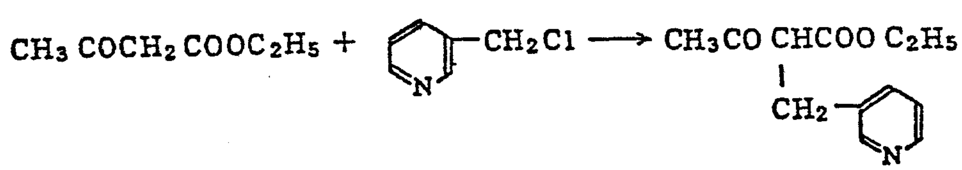

Under nitrogen, 8.4 g of metallic sodium was dissolved in 500 ml of anhydrous ethanol, and after adding 23.8 g of ethyl acetoacetate and stirring the mixture at room temperature for 1 hour, 30 g of 3-chloromethylpyridine hydrochloride was added to the mixture followed by refluxing under mild heating.

Salt formed was filtered away, and the solvent was distilled

away. The residue obtained was purified by column chromatography using chloroform - ethylacetate as a developing solvent to provide 12.0 g of oil product.

NMR ($CDCl_3$)
δ (ppm) ; 1.18 (3H, t)
2.20 (3H, s)
3.13 (2H, d)
3.78 (1H, t)
4.13 (2H, q)
7.04 ~ 7.60 (2H, m)
8.40 (2H, m)
Mass (m/e) 221 ($M^+$)

Examples 41 - 44

By following the same procedure as in Example 40, the following compounds were obtained:

Example 41

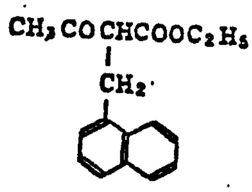

ethyl α-(1-naphthyl-methyl)-acetoacetate

NMR ($CDCl_3$)
δ (ppm) ; 1.16 (3H, t)
2.15 (3H, s)
3.64 (2H, d)
3.95 (1H, t)
4.13 (2H, q)
7.2 ~ 8.2 (7H, m)
Mass (m/e) 270 ($M^+$), 227, 181

Example 42

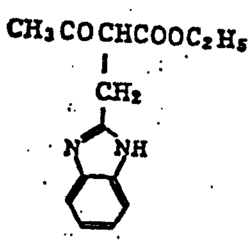

ethyl α-(2-benzimidazolyl-methyl)acetoacetate

Mass (m/e) 260 ($M^+$), 217

Example 43

ethyl α-(p-tert-butylbenzyl)acetoacetate

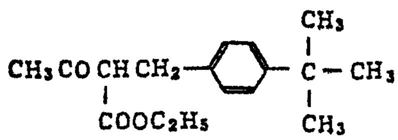

NMR ($CDCl_3$ )
δ(ppm) ; 1.18 (t, 3H)
1.27 (s, 9H)
2.17 (s, 3H)
3.11 (d, 2H)
3.75 (t, 1H)
4.14 (q, 2H)
7.08 (d, 2H)
7.28 (d, 2H)
7.00~7.36 (m, 4H)
IR (neat)
ν ; 1735, 1710 $cm^{-1}$
Mass (m/e) ·
276 ($M^+$), 261 ($M^+$−15)
233 ($M^+$− 43)

Example 44

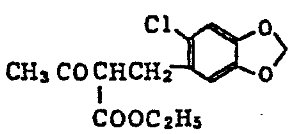

ethyl α-[(6-chloro-1,3-benzodioxole-5-yl)-methyl]acetate

NMR ($CDCl_3$ )
δ (ppm) ; 1.20 (t, 3H)
2.18 (s, 3H)
2.94 ~ 3.35 (m, 2H)
3.60 ~ 3.87 (m, 1H)
4.10 (q, 2H)
5.83 (s, 2H)
6.54 ~ 6.81 (m, 2H)
IR (neat)
ν ; 1735, 1710 $cm^{-1}$
Mass (m/e)
298 ($M^+$), 255 ($M^+$−43)

**CLAIMS:**

1. One or more of the following compounds:

Ethyl α-(3-pyridylmethyl)acetoacetate;
ethyl α-(1-naphthylmethyl)acetoacetate;
ethyl α-(3-pyrazolylmethyl)acetoacetate;
ethyl α-(2-benzimidazolylmethyl)acetoacetate;
ethyl α-(2-N,N-dimethylaminoethyl)acetoacetate;
ethyl α-(3,N-N-dimethylaminopropyl)acetoacetate;
ethyl α-(p-tert-butyl-benzyl)acetoacetate;
ethyl α-(2-quinolylmethyl)acetoacetate;
ethyl α-(p-chlorobenzyl)acetoacetate;
ethyl α-[(6-chloro-1,3-benzodioxole--5-yl)methyl]acetoacetate;
ethyl α-[(1,3-benzodioxole-5-yl)methyl]acetoacetate;
ethyl α-(p-fluorobenzyl)acetoacetate;
ethyl α-phenethylacetoacetate;
ethyl α-[(3,4,5-trimethoxy)benzyl]acetoacetate;
ethyl α-[(p-phenyl)benzyl]acetoacetate;
ethyl α-(p-methylbenzyl)acetoacetate;
ethyl α-n-hexylacetoacetate;
ethyl α-n-octylacetoacetate;
ethyl formacetate;
ethyl α-phenyl formacetate;
ethyl α-n-butyl formacetate;
ethyl α-n-pentyl formacetate;
ethyl α-(3-diethylaminopropyl)acetoacetate;
ethyl α-(2-piperidinoethyl)acetoacetate;
ethyl α-ethylacetoacetate;
ethyl α-propylacetoacetate;
ethyl α-(2-propinyl)acetoacetate;
ethyl α-cyclohexylmethylacetoacetate;
ethyl α-n-butylacetoacetate;
ethyl α-n-pentylacetoacetate;
ethyl α-[(3-methyl)butyl]acetoacetate;
ethyl α-[3-(N,N-dimethylamino)-2-methylpropyl]acetoacetate.

European Patent Office

# EUROPEAN SEARCH REPORT

0180745
Application number

EP 85 11 1879

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| X | CHEMICAL ABSTRACTS, vol. 95, 1981, page 647, abstract no. 97700a, Columbus, Ohio, US; A. KREUTZBERGER et al.: "Tumor inhibitory agents. Part 12. 2-Ureido-4(3H)-pyrimidinones", & ARCH. PHARM. (Weinheim, Ger.) 1981, 314(1), 34-41<br>* Abstract *<br>--- | 1 | C 07 C 123/00<br>C 07 C 101/34<br>C 07 C 69/716<br>C 07 C 69/738<br>C 07 D 239/36<br>C 07 D 213/55<br>C 07 D 231/12<br>C 07 D 235/16<br>C 07 D 215/14<br>C 07 D 317/60<br>C 07 D 317/62 |
| X | EP-A-0 041 492 (HÄSSLE)<br>* Page 23 *<br>--- | 1 | |
| X | DE-A-3 015 738 (C. ERBA)<br>* Page 26 (X) *<br>--- | 1 | |
| X | DE-A-3 017 625 (CHINOIN)<br>* Page 14, example 5; page 24, example 29 *<br>--- | 1 | TECHNICAL FIELDS SEARCHED (Int. Cl.4) |
| X | US-A-4 216 318 (TH.H. BROWN et al.)<br>* Column 5 *<br>--- -/- | 1 | C 07 D 215/00<br>C 07 D 317/00<br>C 07 C 69/00<br>C 07 C 101/00 |

The present search report has been drawn up for all claims

| Place of search | Date of completion of the search | Examiner |
|---|---|---|
| THE HAGUE | 11-02-1986 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

# EUROPEAN SEARCH REPORT

0180745

Application number

EP 85 11 1879

Page 2

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | JOURNAL OF MEDICINAL CHEMISTRY, vol. 18, no. 3, 1975, pages 272-275, Washington, D.C., US; I. KAVAI et al.: "Synthesis and antifolate activity of new diaminopyrimidines and diaminopurines in enzymatic and cellular systems" * Pages 272,274,275 * --- | 1 | |
| X | FR-A-2 144 875 (BOEHRINGER) * Page 10 * --- | 1 | |
| X | US-A-3 666 801 (R.A. ROBINSON) * Columns 1-3 * --- | 1 | |
| X | DE-A-2 835 004 (CHINOIN) * Pages 3,4,17,31-41 * --- | 1 | TECHNICAL FIELDS SEARCHED (Int. Cl.4) |
| X | US-A-4 145 546 (TH.H. BROWN et al.) * Columns 4,5,12 * --- | 1 | |
| X | TETRAHEDRON LETTERS, no. 40, 1973, pages 3895-3898, Pergamon Press, Oxford, GB; T. IPAKTCHI et al.: "Relative configuration of the diacid sidechain of isoharringtonine" * Page 3896 (5) * --- -/- | 1 | |

The present search report has been drawn up for all claims

| Place of search | Date of completion of the search | Examiner |
|---|---|---|
| THE HAGUE | 11-02-1986 | FRANCOIS J.C.L. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

0180745
Application number

EP 85 11 1879

## DOCUMENTS CONSIDERED TO BE RELEVANT



| Category | Citation of document with indication, where appropriate, of relevant passages | Relevant to claim | CLASSIFICATION OF THE APPLICATION (Int. Cl.4) |
|---|---|---|---|
| X | JOURNAL OF THE CHEMICAL SOCIETY, PERKIN TRANS. II, no. 12, 1976, pages 1367-1370, London, GB; R.P. BELL et al.: "Brönsted exponents in the proton abstraction from derivatives of ethyl alpha-benzylacetoacetate, 3-benzylpentane-2,4-dione, and benzylmalononitrile" * Pages 1368,1370 * | 1 | |
| X | CHEMICAL ABSTRACTS, vol. 87, 1977, page 676, abstract no. 200923m, Columbus, Ohio, US; & JP - A - 77 83 717 (YAMANOUCHI PHARMACEUTICAL CO., LTD.) 12-07-1977 * Abstract * | 1 | |
| X | CHEMICAL ABSTRACTS, vol. 93, 1980, page 624, abstract no. 167685j, Columbus, Ohio, US; & JP - A - 80 15 420 (MEIJI SEIKA KAISHA, LTD.) 02-02-1980 * Abstract * | 1 | TECHNICAL FIELDS SEARCHED (Int. Cl.4) |

--- -/-

The present search report has been drawn up for all claims

| Place of search | Date of completion of the search | Examiner |
|---|---|---|
| THE HAGUE | 11-02-1986 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

0180745

Application number

EP 85 11 1879

Page 4

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| X | CHEMICAL ABSTRACTS, vol. 90, 1979, page 620, abstract no. 121337g, Columbus, Ohio, US; C.H. BRIESKORN et al.: "Synthesis of derivatives of indoles, diindolylmethanes and indolyl(tetrahydroindolyl)methanes", & ARCH. PHARM. (Weinheim, Ger.) 1978, 311(11),954-60 * Abstract * | 1 | |
| | --- | | |
| X | CHEMICAL ABSTRACTS, vol. 94, 1981, page 676, abstract no. 83576g, Columbus, Ohio, US; V.V. KUL'GANEK et al.: "General method for the synthesis of ethyl 5-(diethylamino)-2-acetyl(or carbethoxy)-3-R-pentanoates", & IZV. AKAD. NAUK SSSR, SER. KHIM. 1980, (9), 2161-4 * Abstract * | 1 | TECHNICAL FIELDS SEARCHED (Int. Cl.4) |
| | ----- | | |

The present search report has been drawn up for all claims

| Place of search | Date of completion of the search | Examiner |
|---|---|---|
| THE HAGUE | 11-02-1986 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82